# FASCICULE DE BREVET EUROPEEN

(11) **EP 1 013 338 B1**
(45) Date de publication et mention de la délivrance du brevet: **10.10.2001**
(21) Numéro de dépôt: 99402856.1
(22) Date de dépôt: 17.11.1999
(51) Int. Cl.: B01F 17/00, A61K 7/00

(54) **Nanoémulsion à base d'esters gras d'acide phosphorique, et ses utilisations dans les domaines cosmétique, dermatologique, pharmaceutique et/ou ophtalmologique**
Nanoemulsion aus Fettsäureestern von Phosphorsäure und deren Verwendung in Kosmetika, Dermatologie, Pharmazeutika und/oder Ophthalmologie
Nanoemulsion based on fatty esters of phosphoric acid and uses thereof in the fields of cosmetics, dermatology, pharmaceuticals and/or ophthalmology

(30) Priorité: 23.12.1998 FR 9816370
(43) Date de publication de la demande: 28.06.2000
(73) Titulaire: L'OREAL, 75008 Paris (FR)
(72) Inventeur: Simmonet, Jean-Thierry, 75011 Paris (FR); Legret, Sylvie, 92320 Chatillon (FR); Sonneville, Odile, 75014 Paris (FR)
(74) Mandataire: Rasson, Catherine

(56) Documents cités:
- EP-A- 0 842 652
- EP-A- 0 852 941

## Description

La présente invention concerne une nanoémulsion à base d'un tensioactif anionique choisi parmi les esters gras d'acide phosphorique et leurs dérivés oxyéthylénés, et d'au moins une huile ayant un poids moléculaire supérieur à 400, le rapport en poids de la quantité de phase huileuse sur la quantité de tensioactif allant de 2 à 10.

L'invention se rapporte aussi au procédé de préparation de la dite nanoémulsion et à ses utilisations notamment dans les domaines cosmétique, dermatologique et/ou ophtalmologique. Cette nanoémulsion est stable au stockage et peut contenir des quantités importantes d'huile tout en conservant une bonne transparence et en ayant de bonnes propriétés cosmétiques.

Les nanoémulsions sont des émulsions huile-dans-eau dont les globules d'huile ont une granulométrie très fine, c'est-à-dire une taille moyenne en nombre, inférieure à 100 nm. Elles sont généralement fabriquées par fragmentation mécanique d'une phase huileuse dans une phase aqueuse en présence de tensioactif. Dans le cas des nanoémulsions, la très petite taille des globules huileux est obtenue notamment grâce à au moins un passage dans un homogénéiseur haute-pression. La petite taille des globules leur confère des propriétés intéressantes sur le plan cosmétique, qui les distinguent des émulsions classiques : elles sont transparentes et présentent une texture originale. Elles peuvent également véhiculer les actifs de façon plus efficace.

On connaît dans l'état de la technique des microémulsions transparentes. Les microémulsions ne sont pas à proprement parler des émulsions, contrairement aux nanoémulsions ; ce sont des solutions transparentes de micelles gonflées par de l'huile qui est en général à chaîne très courte (ex : hexane, décane) et qui est solubilisée grâce à la présence conjointe d'une quantité importante de tensioactifs et de co-tensioactifs formant les micelles. La taille des micelles gonflées est très petite en raison de la faible quantité d'huile qu'elles peuvent solubiliser. Cette très petite taille des micelles est la cause de leur transparence comme pour les nanoémulsions. Cependant, contrairement aux nanoémulsions, les microémulsions se forment spontanément par mélange des constituants sans apport d'énergie mécanique autre qu'une simple agitation magnétique. Les inconvénients majeurs des microémulsions sont liés à leur forte proportion en tensioactifs, conduisant à des intolérances et entraînant un toucher collant lors de l'application sur la peau. Par ailleurs, leur domaine de formulation est en général très étroit et leur stabilité en température très limitée.

En outre, on connaît dans l'état de la technique des nanoémulsions comprenant une phase lipidique amphiphile constituée de phospholipides, d'eau et d'huile. Ces émulsions présentent l'inconvénient d'être instables au stockage aux températures traditionnelles de conservation, à savoir entre 0 et 45°C. Elles conduisent à des compositions jaunes et produisent des odeurs de rance qui se développent après quelques jours de conservation.

On connaît aussi des nanoémulsions stabilisées par un enrobage cristal liquide lamellaire obtenu par l'association d'un tensioactif hydrophile et d'un tensioactif lipophile. Toutefois, ces associations sont délicates à déterminer. De plus, les nanoémulsions obtenues présentent un toucher cireux et filmogène peu agréable pour l'utilisateur.

Par ailleurs, le document EP-A-728 460 décrit des nanoémulsions à base de lipides amphiphiles non-ioniques fluides. Toutefois, ces nanoémulsions présentent l'inconvénient d'avoir un effet collant lors de l'application sur la peau.

Il subsiste donc le besoin de nanoémulsions n'ayant ni les inconvénients de celles de l'art antérieur ni les inconvénients des microémulsions.

La demanderesse a maintenant découvert, de façon inattendue, que l'utilisation d'un tensioactif anionique choisi parmi les esters gras d'acide phosphorique et leurs dérivés oxyéthylénés, et d'au moins une huile ayant un poids moléculaire supérieur à 400 (= 400 grammes par mole) permettait d'obtenir de nouvelles nanoémulsions présentant tous les avantages des nanoémulsions connues sans leurs inconvénients.

La présente invention a pour objet une nanoémulsion comportant une phase huileuse dispersée dans une phase aqueuse et ayant des globules d'huile dont la taille moyenne en nombre est inférieure à 100 nm, caractérisée en ce qu'elle contient au moins un tensioactif anionique choisi parmi les esters gras d'acide phosphorique et leurs dérivés oxyéthylénés, et au moins une huile ayant un poids moléculaire supérieur à 400, et en ce que le rapport en poids de la quantité de phase huileuse sur la quantité de tensioactif va de 2 à 10.

Les nanoémulsions selon l'invention ont généralement un aspect transparent à bleuté. Leur transparence se mesure par un coefficient de transmittance à 600 nm allant de 10 à 90 % ou bien par une turbidité allant de 60 à 600 NTU et de préférence de 70 à 300 NTU, turbidité mesurée au turbidimètre portatif HACH-Modèle 2100 P.

Les globules d'huile des nanoémulsions de l'invention ont une taille moyenne en nombre, inférieure à 100 nm et de préférence allant de 20 à 75 nm et plus préférentiellement de 40 à 60 nm. La diminution de la taille des globules permet de favoriser la pénétration des actifs dans les couches superficielles de la peau (effet véhicule).

Le tensioactif anionique utilisable dans la nanoémulsion de l'invention est choisi parmi les esters gras d'acide phosphorique, les dérivés oxyéthylénés de ces esters, leurs sels et leurs mélanges. Selon un mode particulier de réalisation de l'invention, la nanoémulsion de l'invention est exempte de tout tensioactif autre que les esters gras d'acide phosphorique et/ou leurs dérivés oxyéthylénés.

Les esters gras d'acide phosphorique, et leurs dérivés oxyéthylénés, utilisables comme tensioactifs dans la nanoémulsion selon l'invention peuvent être choisis notamment dans le groupe comprenant les esters formés d'acide phosphorique et d'au moins un alcool comportant une chaîne alkyle linéaire ou ramifiée, saturée ou non saturée, ayant de 8 à 22 atomes de carbone et les esters formés d'acide phosphorique et d'au moins un alcool éthoxylé, comportant une chaîne alkyle linéaire ou ramifiée, saturée ou non saturée, ayant de 8 à 22 atomes de carbone et comportant de 2 à 40 groupes oxyéthylénés, leurs sels et leurs mélanges. On peut en effet utiliser un mélange d'un ou plusieurs de ces esters d'acide phosphorique dans la nanoémulsion de l'invention.

Ces esters peuvent être notamment choisis parmi les esters d'acide phosphorique et d'alcools en C9-C15 ou leurs sels, tels que le sel de potassium d'alkyl-C9-15-phosphate commercialisé sous la dénomination Arlatone MAP par la société ICI, les esters d'acide phosphorique et d'alcools stéarylique et/ou isostéarylique, tels que le phosphate d'alcools stéarylique/isostéarylique (nom CTFA : Octyldecyl phosphate), commercialisé sous la dénomination Hostaphat CG120 par la société Hoechst Celanese, les esters d'acide phosphorique et d'alcool cétylique, et leurs dérivés oxyéthylénés tels que le produit commercialisé sous la dénomination Crodafos CES (mélange d'alcool cétéarylique, de dicétyl phosphate et de ceteth-10 phosphate) par la société Croda, les esters d'acide phosphorique et d'alcool tridécylique, et leurs dérivés oxyéthylénés tels que le produit commercialisé sous la dénomination Crodafos T10 (nom CTFA : Trideceth-10 Phosphate) par la société Croda. Les dérivés oxyéthylénés d'acide phosphorique et d'alcool gras peuvent être préparés conformément à la description donnée dans la demande de brevet WO-A-96/14145.

Les esters gras d'acide phosphorique, utilisés comme tensioactifs sont de préférence employés sous forme neutralisée à un pH d'environ 7, l'agent de neutralisation étant choisi parmi les bases inorganiques telles que la soude, la potasse, l'ammoniac, et les bases organiques telles que la mono-, di- et tri-éthanolamine, l'aminométhylpropanediol-1,3, la N-méthylglucamine, les acides aminés basiques comme l'arginine et la lysine, et leurs mélanges.

La quantité de tensioactif dans la nanoémulsion de l'invention peut aller par exemple de 0,2 à 15 % en poids et de préférence de 1 à 8 % en poids par rapport au poids total de la nanoémulsion.

Le rapport en poids de la quantité de la phase huileuse sur la quantité de tensioactif va de 2 à 10 et, de préférence, de 3 à 6. On entend ici par "quantité de phase huileuse" la quantité totale des constituants de cette phase sans inclure la quantité de tensioactif.

La nanoémulsion selon l'invention contient au moins une huile de poids moléculaire supérieur à 400. Les huiles de poids moléculaire supérieur à 400 peuvent être choisies parmi les huiles d'origine animale ou végétale, les huiles minérales, les huiles de synthèse et les huiles de silicone, et leurs mélanges. Comme huiles de ce type, on peut citer par exemple le palmitate d'isocétyle, le stéarate d'isocétyle, l'huile d'avocat, l'huile de jojoba.

En outre, la phase huileuse peut éventuellement contenir d'autres huiles et notamment des huiles ayant un poids moléculaire inférieur à 400. Ces huiles sont choisies également parmi les huiles d'origine animale ou végétale, les huiles minérales, les huiles de synthèse et les huiles de silicone. On peut citer par exemple comme huiles de poids moléculaire inférieur à 400, l'isododécane, l'isohexadécane, les huiles de silicone volatiles, le myristate d'isopropyle, le palmitate d'isopropyle, l'isoparaffine C11-C13.

La phase huileuse peut contenir également des corps gras autres que les huiles indiquées ci-dessus, tels que les alcools gras comme les alcools stéarylique, cétylique, béhénique, les acides gras comme les acides stéarique, palmitique et béhénique, les huiles fluorées, les cires, les gommes et leurs mélanges.

Les nanoémulsions conformes à l'invention comportent une quantité de phase huileuse allant de préférence de 2 à 40 % et mieux de 5 à 30 % en poids par rapport au poids total de la nanoémulsion, la proportion d'huile(s) ayant un poids moléculaire supérieur à 400 représentant de préférence au moins 40 % en poids de la phase huileuse.

Selon une forme particulière de réalisation de l'invention, la nanoémulsion de l'invention contient en outre un ou plusieurs lipides amphiphiles ioniques.

Les lipides amphiphiles ioniques pouvant être utilisés dans les nanoémulsions de l'invention sont choisis de préférence dans le groupe formé par les lipides amphiphiles anioniques et les dérivés alkylsulfoniques.

Les lipides amphiphiles anioniques peuvent être plus particulièrement choisis dans-le groupe formé par
- les sels alcalins du dicetyl- et du dimyristylphosphate ;
- les sels alcalins du cholestérol sulfate ;
- les sels alcalins du cholestérol phosphate ;
- les lipoaminoacides et leurs sels tels que les acylglutamates mono- et di-sodiques comme le sel disodique de l'acide N-stéaroyl L-glutamique commercialisé sous la dénomination Acylglutamate HS21 par la société AJINOMOTO ;
- les sels de sodium de l'acide phosphatidique ;
- les phospholipides.

Les dérivés alkylsulfoniques peuvent être plus particulièrement choisis parmi les dérivés alkylsulfoniques de formule (I) suivante : 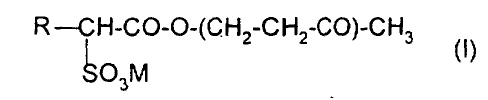 dans laquelle R représente un radical alkyle comportant de 16 à 22 atomes de carbone, en particulier les radicaux C₁₆H₃₃ et C₁₈H₃₇ pris en mélange ou séparément, et M est un métal alcalin tel que le sodium.

Selon un mode préféré de réalisation de l'invention, on utilise comme lipide amphiphile ionique un lipoaminoacide.

Les lipides ioniques amphiphiles peuvent être introduits dans l'une ou l'autre phase de la nanoémulsion. Lorsqu'ils sont présents dans la nanoémulsion de l'invention, ils peuvent être utilisés dans des concentrations allant de préférence de 0,01 à 5 % en poids et plus particulièrement de 0,25 à 1 % en poids par rapport au poids total de la nanoémulsion.

Les émulsions conformes à la présente invention peuvent contenir des additifs pour améliorer la transparence de la formulation.

Ces additifs sont choisis de préférence dans le groupe formé par :
- les alcools inférieurs comportant de 1 à 8 atomes de carbone et plus particulièrement de 2 à 6 atomes de carbone, tels que l'éthanol ;
- les glycols tels que la glycérine, le propylène glycol, le 1,3- butylène glycol, le dipropylène glycol, le pentylène glycol, l'isoprène glycol et les polyéthylèneglycols comportant de 4 à 16 et de préférence de 8 à 12 unités d'oxyde d'éthylène ;
- les sucres tels que le glucose, le fructose, le maltose, le lactose, le sucrose.

Ces additifs peuvent être utilisés en mélange. Lorsqu'ils sont présents dans la nanoémulsion de l'invention, ils peuvent être utilisés à des concentrations allant de préférence de 0,01 à 30 % en poids par rapport au poids total de la nanoémulsion, et mieux de 5 à 20 % en poids par rapport au poids total de la nanoémulsion. La quantité d'alcool(s) et/ou de sucre(s) va de préférence de 5 à 20 % en poids par rapport au poids total de la nanoémulsion et la quantité de glycol(s) va de préférence de 5 à 15 % en poids par rapport au poids total de la nanoémulsion.

En outre, l'utilisation des alcools tels que définis ci-dessus, à des concentrations supérieures ou égales à 15 % en poids permet d'obtenir des émulsions sans conservateur.

Les nanoémulsions définies ci-dessus peuvent être utilisées dans tout domaine où ce type de composition est utile. Elles peuvent constituer notamment des compositions à usage topique et notamment cosmétiques ou dermatologiques. Elles peuvent aussi être utilisées comme supports ophtalmiques. Elles peuvent en outre constituer dans le domaine pharmaceutique une composition pharmaceutique qui peut être administrée par voie orale, parentérale ou transcutanée.

Un autre objet de l'invention consiste donc en une composition à usage topique, caractérisée en ce qu'elle contient une nanoémulsion telle que définie précédemment.

Une composition à usage topique ou pharmaceutique contient un milieu physiologiquement acceptable, c'est-à-dire compatible avec la peau, les muqueuses, le cuir chevelu, les yeux et/ou les cheveux.

L'invention a aussi pour objet un support ophtalmique, caractérisé en ce qu'il contient une nanoémulsion telle que définie précédemment.

L'invention a aussi pour objet une composition pharmaceutique, caractérisée en ce qu'elle contient une nanoémulsion telle que définie précédemment.

Les nanoémulsions de l'invention peuvent contenir des actifs hydrosolubles ou liposolubles ayant une activité cosmétique, dermatologique ou ophtalmique. Les actifs liposolubles sont dans les globules huileux de l'émulsion, tandis que les actifs hydrosolubles sont dans la phase aqueuse de l'émulsion. On peut citer, à titre d'exemples d'actifs, les vitamines telles que la vitamine E, et leurs dérivés et en particulier leurs esters, les provitamines telles que le panthénol, les humectants et les filtres solaires.

Comme actifs ophtalmiques, on peut citer par exemple les agents anti-glaucome, tels que le betaxolol ; les antibiotiques tels que l'acyclovir ; les antiallergiques ; les agents anti-inflammatoires tels que l'ibuprofène et ses sels, le diclofénac et ses sels, l'indométhacine ; les agents antiviraux.

Les nanoémulsions conformes à l'invention peuvent se présenter sous forme de lotion, de sérum, de crème, de lait ou d'eau de toilette et peuvent contenir des adjuvants habituellement utilisés dans les domaines cosmétique, dermatologique et ophtalmique, tels que par exemple les gélifiants, les conservateurs, les antioxydants et les parfums. Elles peuvent aussi se présenter sous forme de collyre, en particulier pour les applications ophtalmologiques.

Parmi les gélifiants utilisables, on peut citer les dérivés de cellulose, les dérivés d'algues, les gommes naturelles et les polymères synthétiques tels que les polymères et copolymères d'acides carboxyvinyliques comme ceux commercialisés sous la dénomination CARBOPOL par la société GOODRICH.

L'invention a aussi pour objet un procédé de préparation d'une nanoémulsion telle que définie ci-dessus, ce procédé consistant à mélanger la phase aqueuse et la phase huileuse sous agitation vive, à une température allant de 10 à 80°C, puis à effectuer une homogénéisation à une pression allant de préférence de 6.10⁷ Pa à 18.10⁷ Pa (homogénéisation haute pression). Le cisaillement va de préférence de 2.10⁶ s⁻¹ à 5.10⁸ s⁻¹ et mieux de 1.10⁸ s⁻¹ à 3.10⁸ s⁻¹ (s⁻¹ signifie seconde⁻¹).

La nanoémulsion de l'invention peut être par exemple utilisée pour le soin, le traitement, le maquillage de la peau, du visage et/ou du cuir chevelu.

L'invention a donc aussi pour objet l'utilisation cosmétique de la nanoémulsion telle que définie ci-dessus pour le soin, le traitement et/ou le maquillage de la peau, du visage et/ou du cuir chevelu.

En outre, la nanoémulsion de l'invention peut aussi être utilisée pour le soin et/ou le traitement des cheveux. Elle permet d'obtenir un dépôt d'huile sur les cheveux, ce qui rend ceux-ci plus brillants, plus résistants au coiffage, sans toutefois les alourdir. Elle permet aussi, en prétraitement, d'améliorer les effets de la coloration ou de la permanente.

L'invention a donc aussi pour objet l'utilisation cosmétique de la nanoémulsion telle que définie ci-dessus pour le soin et/ou le traitement des cheveux.

La nanoémulsion selon l'invention permet notamment une bonne hydratation de la peau, des muqueuses et/ou du cuir chevelu, et est particulièrement adaptée au traitement de la peau sèche.

Un autre objet de l'invention est donc un procédé cosmétique de soin et/ou d'hydratation de la peau, des muqueuses et/ou du cuir chevelu, caractérisé en ce qu'on applique sur la peau, les muqueuses et/ou le cuir chevelu, une nanoémulsion telle que définie ci-dessus.

L'invention porte également sur l'utilisation de la nanoémulsion selon l'invention pour la fabrication d'une composition dermatologique destinée au traitement de la peau sèche.

Enfin, l'invention porte aussi sur l'utilisation de la nanoémulsion selon l'invention pour la fabrication d'une composition ophtalmologique.

L'exemple qui suit permettra de mieux comprendre l'invention, sans toutefois présenter un caractère limitatif. Les quantités indiquées sont en % en poids.

### Exemple : Fluide démaquillant

### Phase huileuse :

- Sel disodique de l'acide N-Stéaroyl L-glutamique (Acylglutamate HS21 de la société AJINOMOTO) 0,5 %
- Stéarate d'isocétyle (P.M. = 508) 10 %
- Palmitate d'isopropyle (P.M. = 298) 5 %

### Phase aqueuse :

- Hostaphat CG120 (Société Hoechst Celanese) 4,5 %
- NaOH(1N) 5 %
- Glycérine 5 %
- Dipropylène glycol 10 %
- Eau 60 %

On obtient une nanoémulsion transparente dont la taille des globules est de 57 nm et la turbidité de 250 NTU.

## Revendications

1. Nanoémulsion comportant une phase huileuse dispersée dans une phase aqueuse et ayant des globules d'huile dont la taille moyenne en nombre est inférieure à 100 nm, **caractérisée en ce qu'**elle contient au moins un tensioactif anionique choisi parmi les esters gras d'acide phosphorique et leurs dérivés oxyéthylénés, et au moins une huile ayant un poids moléculaire supérieur à 400, et **en ce que** le rapport en poids de la quantité de phase huileuse sur la quantité de tensioactif va de 2 à 10.

2. Nanoémulsion selon la revendication 1, **caractérisée en ce qu'**elle a une turbidité allant de 60 à 600 NTU.

3. Nanoémulsion selon la revendication 1 ou 2, **caractérisée en ce que** la quantité de tensioactif va de 0,2 à 15 % en poids et de préférence de 1 à 8 % en poids par rapport au poids total de la nanoémulsion.

4. Nanoémulsion selon l'une quelconque des revendications précédentes, **caractérisée en ce que** le rapport en poids de la quantité de phase huileuse sur la quantité de tensioactif va de 3 à 6.

5. Nanoémulsion selon l'une quelconque des revendications précédentes, **caractérisée en ce que** les globules d'huile ont une taille moyenne allant de 20 à 75 nm.

6. Nanoémulsion selon l'une quelconque des revendications précédentes, **caractérisée en ce que** le tensioactif est choisi dans le groupe comprenant les esters formés d'acide phosphorique et d'au moins un alcool comportant une chaîne alkyle linéaire ou ramifiée, saturée ou non saturée, ayant de 8 à 22 atomes de carbone et les esters formés d'acide phosphorique et d'au moins un alcool éthoxylé, comportant une chaîne alkyle linéaire ou ramifiée, saturée ou non saturée, ayant de 8 à 22 atomes de carbone et comportant de 2 à 40 groupes oxyéthylénés, leurs sels et leurs mélanges.

7. Nanoémulsion selon l'une quelconque des revendications précédentes, **caractérisée en ce que** le tensioactif est choisi dans le groupe comprenant les esters d'acide phosphorique et d'alcools en C9-C15, les esters d'acide phosphorique et d'alcools stéarylique et/ou isostéarylique, les esters d'acide phosphorique et d'alcool cétylique, et leurs dérivés oxyéthylénés, les esters d'acide phosphorique et d'alcool tridécylique, et leurs dérivés oxyéthylénés, leurs sels et leurs mélanges.

8. Nanoémulsion selon l'une quelconque des revendications précédentes, **caractérisée en ce qu'**elle contient au moins un agent de neutralisation choisi parmi les bases inorganiques, les bases organiques et leurs mélanges.

9. Nanoémulsion selon l'une quelconque des revendications précédentes, **caractérisée en ce que** l'huile de poids moléculaire supérieur à 400 est choisie parmi les huiles d'origine animale ou végétale, les huiles minérales, les huiles de synthèse et les huiles de silicone, et leurs mélanges.

10. Nanoémulsion selon l'une quelconque des revendications précédentes, **caractérisée en ce que** la phase huileuse contient en outre au moins une huile ayant un poids moléculaire inférieur à 400.

11. Nanoémulsion selon l'une quelconque des revendications précédentes, **caractérisée en ce que** la phase huileuse contient au moins 40 % en poids d'huile(s) ayant un poids moléculaire supérieur à 400, par rapport au poids total de la phase huileuse.

12. Nanoémulsion selon l'une quelconque des revendications précédentes, **caractérisée en ce que** la quantité de phase huileuse va de 2 à 40 % en poids par rapport au poids total de la nanoémulsion.

13. Nanoémulsion selon l'une quelconque des revendications précédentes, **caractérisée en ce qu'**elle contient en outre au moins un lipide amphiphile ionique choisi dans le groupe formé par les lipides amphiphiles anioniques, les dérivés alkylsulfoniques.

14. Nanoémulsion selon la revendication 13, **caractérisée en ce que** les lipides amphiphiles ioniques sont choisis dans le groupe formé par
- les sels alcalins du dicetyl- et du dimyristylphosphate ;
- les sels alcalins du cholestérol sulfate ;
- les sels alcalins du cholestérol phosphate ;
- les sels de lipoaminoacides ;
- les sels de sodium de l'acide phosphatidique ;
- les phospholipides ;
- les dérivés alkylsulfoniques de formule (I) : 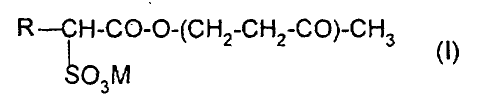 dans laquelle R représente des radicaux alkyle en C₁₆-C₂₂ pris en mélange ou séparément et M est un métal alcalin ;
et leurs mélanges.

15. Nanoémulsion selon la revendication 13 ou 14, **caractérisée en ce que** la quantité de lipide(s) amphiphile(s) ionique(s) va de 0,01 à 5 % en poids par rapport au poids total de la nanoémulsion.

16. Nanoémulsion selon l'une quelconque des revendications précédentes, **caractérisée en ce qu'**elle contient un additif permettant d'améliorer la transparence, choisi parmi les alcools inférieurs, les glycols, les sucres et leurs mélanges.

17. Nanoémulsion selon la revendication précédente, **caractérisée en ce que** l'additif est présent en une concentration allant de 5 à 20 % en poids par rapport au poids total de la nanoémulsion.

18. Nanoémulsion selon l'une quelconque des revendications précédentes, **caractérisée en ce qu'**elle contient un actif cosmétique, dermatologique ou ophtalmologique.

19. Composition à usage topique, **caractérisée en ce qu'**elle contient une nanoémulsion selon l'une quelconque des revendications 1 à 18.

20. Support ophtalmique, **caractérisé en ce qu'**il contient une nanoémulsion selon l'une quelconque des revendications 1 à 18.

21. Composition pharmaceutique, **caractérisée en ce qu'**elle contient une nanoémulsion selon l'une quelconque des revendications 1 à 18.

22. Utilisation cosmétique de la nanoémulsion selon l'une quelconque des revendications 1 à 18, pour le soin, le traitement et/ou le maquillage de la peau, du visage et/ou du cuir chevelu.

23. Utilisation cosmétique de la nanoémulsion selon l'une quelconque des revendications 1 à 18, pour le soin et/ou le traitement des cheveux.

24. Procédé cosmétique de soin et/ou d'hydratation de la peau, des muqueuses et/ou du cuir chevelu, **caractérisé en ce que** qu'on applique sur la peau, les muqueuses et/ou le cuir chevelu, une nanoémulsion selon l'une quelconque des revendications 1 à 18.

25. Utilisation de la nanoémulsion selon l'une quelconque des revendications 1 à 18, pour la fabrication d'une composition dermatologique destinée au traitement de la peau sèche.

26. Utilisation de la nanoémulsion selon l'une quelconque des revendications 1 à 18, pour la fabrication d'une composition ophtalmologique.

27. Procédé de préparation d'une nanoémulsion selon l'une quelconque des revendications 1 à 18, consistant à mélanger la phase aqueuse et la phase huileuse sous agitation vive, à une température ambiante allant de 10 à 80°C, puis à effectuer une homogénéisation à une pression allant de 6.10⁷ Pa à 18.10⁷ Pa.

28. Procédé selon la revendication précédente, **caractérisé en ce que** le cisaillement va de 2.10⁶ s⁻¹ à 5.10⁸ s⁻¹.

## Patentansprüche

1. Nanoemulsion, die eine in einer wäßrigen Phase dispergierte Ölphase aufweist, wobei das Zahlenmittel der Partikelgröße der Ölkügelchen unter 100 nm liegt, **dadurch gekennzeichnet, daß** sie mindestens einen anionischen grenzflächenaktiven Stoff, der unter den Fettsäureestern von Phosphorsäure und deren ethoxylierten Derivaten ausgewählt ist, und mindestens ein Öl mit einer Molmasse über 400 enthält und das Gewichtsverhältnis der Menge der Ölphase und der Menge des grenzflächenaktiven Stoffes im Bereich von 2 bis 10 liegt.

2. Nanoemulsion nach Anspruch 1, **dadurch gekennzeichnet, daß** sie eine Trübung von 60 bis 600 NTU aufweist.

3. Nanoemulsion nach Anspruch 1 oder 2, **dadurch gekennzeichnet, daß** die Menge des grenzflächenaktiven Stoffes im Bereich von 0,2 bis 15 Gew.-% und vorzugsweise 1 bis 8 Gew.-%, bezogen auf das Gesamtgewicht der Nanoemulsion, liegt.

4. Nanoemulsion nach einem der vorhergehenden Ansprüche, **dadurch gekennzeichnet, daß** das Gewichtsverhältnis der Menge der Ölphase und der Menge des grenzflächenaktiven Stoffes im Bereich von 3 bis 6 liegt.

5. Nanoemulsion nach einem der vorhergehenden Ansprüche, **dadurch gekennzeichnet, daß** die Ölkügelchen eine mittlere Größe von 20 bis 75 nm aufweisen.

6. Nanoemulsion nach einem der vorhergehenden Ansprüche, **dadurch gekennzeichnet, daß** der grenzflächenaktive Stoff unter den Estern von Phosphorsäure und mindestens einem Alkohol, der eine geradkettige oder verzweigte, gesättigte oder ungesättigte Alkylkette mit 8 bis 22 Kohlenstoffatomen aufweist, den Estern von Phosphorsäure und mindestens einem ethoxylierten Alkohol, der eine geradkettige oder verzweigte, gesättigte oder ungesättigte Alkylkette mit 8 bis 22 Kohlenstoffatomen und 2 bis 40 ethoxylierte Gruppen aufweist, deren Salzen und deren Gemischen ausgewählt ist.

7. Nanoemulsion nach einem der vorhergehenden Ansprüche, **dadurch gekennzeichnet, daß** der grenzflächenaktive Stoff unter den Estern von Phosphorsäure und C₉₋₁₅-Alkoholen, Estern von Phosphorsäure und Stearyl- und/oder Isostearylalkohol, Estern von Phosphorsäure und Cetylalkohol und deren ethoxylierten Derivaten, Estern von Phosphorsäure und Tridecylalkohol und deren ethoxylierten Derivaten und deren Salzen und Gemischen ausgewählt ist.

8. Nanoemulsion nach einem der vorhergehenden Ansprüche, **dadurch gekennzeichnet, daß** sie mindestens ein Neutralisierungsmittel enthält, das unter den anorganischen Basen, organischen Basen und deren Gemischen ausgewählt ist.

9. Nanoemulsion nach einem der vorhergehenden Ansprüche, **dadurch gekennzeichnet, daß** das Öl mit einer Molmasse über 400 unter den Ölen tierischer oder pflanzlicher Herkunft, Mineralölen, synthetischen Ölen und Siliconölen und deren Gemischen ausgewählt ist.

10. Nanoemulsion nach einem der vorhergehenden Ansprüche, **dadurch gekennzeichnet, daß** die Ölphase auch mindestens ein Öl mit einer Molmasse unter 400 enthält.

11. Nanoemulsion nach einem der vorhergehenden Ansprüche, **dadurch gekennzeichnet, daß** die Ölphase mindestens 40 Gew.-% Öl(e) mit einer Molmasse über 400, bezogen auf das Gesamtgewicht der Ölphase, enthält.

12. Nanoemulsion nach einem der vorhergehenden Ansprüche, **dadurch gekennzeichnet, daß** der Mengenanteil der Ölphase im Bereich von 2 bis 40 Gew.-%, bezogen auf das Gesamtgewicht der Nanoemulsion, liegt.

13. Nanoemulsion nach einem der vorhergehenden Ansprüche, **dadurch gekennzeichnet, daß** sie ferner mindestens ein ionisches amphiphiles Lipid enthält, das unter den anionischen amphiphilen Lipiden und den Alkylsulfonsäurederivaten ausgewählt ist.

14. Nanoemulsion nach Anspruch 13, **dadurch gekennzeichnet, daß** die ionischen amphiphilen Lipide ausgewählt sind unter:
- den Alkalisalzen von Dicetyl- und Dimyristylphosphat;
- den Alkalisalzen von Cholesterinsulfat;
- den Alkalisalzen von Cholesterinphosphat;
- den Salzen von Lipoaminosäuren;
- den Natriumsalzen von Phosphatidsäure;
- den Phospholipiden; und
- den Alkylsulfonsäurederivaten der Formel (I): 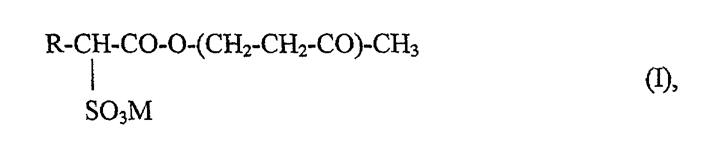 worin R als Gemisch oder einzeln Alkylgruppen mit 16 bis 22 Kohlenstoffatomen bedeutet und M ein Alkalimetall ist.

15. Nanoemulsion nach Anspruch 13 oder 14, **dadurch gekennzeichnet, daß** die Menge des oder der anionischen amphiphilen Lipide im Bereich von 0,01 bis 5 Gew.-%, bezogen auf das Gesamtgewicht der Nanoemulsion, liegt.

16. Nanoemulsion nach einem der vorhergehenden Ansprüche, **dadurch gekennzeichnet, daß** sie einen Zusatzstoff enthält, der die Transparenz verbessern kann und unter den niederen Alkoholen, Glykolen, Zuckern und deren Gemischen ausgewählt ist.

17. Nanoemulsion nach dem vorhergehenden Anspruch, **dadurch gekennzeichnet, daß** der Zusatzstoff in einer Konzentration von 5 bis 20 Gew.-%, bezogen auf das Gesamtgewicht der Nanoemulsion, vorliegt.

18. Nanoemulsion nach einem der vorhergehenden Ansprüche, **dadurch gekennzeichnet, daß** sie einen kosmetischen, dermatologischen oder ophthalmologischen Wirkstoff enthält.

19. Zusammensetzung zur topischen Anwendung, **dadurch gekennzeichnet, daß** sie eine Nanoemulsion nach einem der Ansprüche 1 bis 18 enthält.

20. Ophfhalmischer Träger, **dadurch gekennzeichnet, daß** er eine Nanoemulsion nach einem der Ansprüche 1 bis 18 enthält.

21. Pharmazeutische Zusammensetzung, **dadurch gekennzeichnet, daß** sie eine Nanoemulsion nach einem der Ansprüche 1 bis 18 enthält.

22. Kosmetische Verwendung der Nanoemulsion nach einem der Ansprüche 1 bis 18 zur Pflege, zur Behandlung und/oder zum Schminken der Haut, des Gesichts und/oder der Kopfhaut.

23. Kosmetische Verwendung der Nanoemulsion nach einem der Ansprüche 1 bis 18 zur Pflege und/oder Behandlung der Haare.

24. Kosmetisches Verfahren zur Pflege und/oder Hydratisierung der Haut, der Schleimhäute und/oder der Kopfhaut, **dadurch gekennzeichnet, daß** auf die Haut, die Schleimhäute und/oder die Kopfhaut eine Nanoemulsion nach einem der Ansprüche 1 bis 18 aufgetragen wird.

25. Verwendung der Nanoemulsion nach einem der Ansprüche 1 bis 18 zur Herstellung einer dermatologischen Zusammensetzung, die zur Behandlung von trockener Haut vorgesehen ist.

26. Verwendung der Nanoemulsion nach einem der Ansprüche 1 bis 18 zur Herstellung einer ophthalmologischen Zusammensetzung.

27. Verfahren zur Herstellung einer Nanoemulsion nach einem der Ansprüche 1 bis 18, das darin besteht, die wäßrige Phase und die Ölphase unter kräftigem Rühren bei einer Temperatur von 10 bis 80 °C zu vermischen und dann eine Homogenisierung bei einem Druck von 6 · 10⁷ bis 18 · 10⁷ Pa durchzuführen.

28. Verfahren nach dem vorhergehenden Anspruch, **dadurch gekennzeichnet, daß** die Scherbeanspruchung im Bereich von 2 · 10⁶ bis 5 · 10⁸ s⁻¹ liegt.

## Claims

1. Nanoemulsion comprising an oily phase dispersed in an aqueous phase and having oil globules with a number-average size of less than 100 nm, **characterized in that** it comprises at least one anionic surfactant chosen from phosphoric acid fatty esters and their oxyethylenated derivatives and at least one oil having a molecular weight of greater than 400 and **in that** the ratio by weight of the amount of oily phase to the amount of surfactant ranges from 2 to 10.

2. Nanoemulsion according to Claim 1, **characterized in that** it has a turbidity ranging from 60 to 600 NTU.

3. Nanoemulsion according to Claim 1 or 2, **characterized in that** the amount of surfactant ranges from 0.2 to 15% by weight and preferably from 1 to 8% by weight with respect to the total weight of the nanoemulsion.

4. Nanoemulsion according to any one of the preceding claims, **characterized in that** the ratio by weight of the amount of oily phase to the amount of surfactant ranges from 3 to 6.

5. Nanoemulsion according to any one of the preceding claims, **characterized in that** the oil globules have an average size ranging from 20 to 75 nm.

6. Nanoemulsion according to any one of the preceding claims, **characterized in that** the surfactant is chosen from the group consisting of the esters formed from phosphoric acid and from at least one alcohol comprising a saturated or unsaturated, linear or branched alkyl chain having from 8 to 22 carbon atoms and the esters formed from phosphoric acid and from at least one ethoxylated alcohol comprising a saturated or unsaturated, linear or branched alkyl chain having from 8 to 22 carbon atoms and comprising from 2 to 40 oxyethylene groups, their salts and their mixtures.

7. Nanoemulsion according to any one of the preceding claims, **characterized in that** the surfactant is chosen from the group consisting of the esters of phosphoric acid and of C₉-C₁₅ alcohols, the esters of phosphoric acid and of stearyl and/or isostearyl alcohols, the esters of phosphoric acid and of cetyl alcohol and their oxyethylenated derivatives, the esters of phosphoric acid and of tridecyl alcohol and their oxyethylenated derivatives, their salts and their mixtures.

8. Nanoemulsion according to any one of the preceding claims, **characterized in that** it comprises at least one neutralization agent chosen from inorganic bases, organic bases and their mixtures.

9. Nanoemulsion according to any one of the preceding claims, **characterized in that** the oil with a molecular weight of greater than 400 is chosen from oils of animal or vegetable origin, mineral oils, synthetic oils and silicone oils, and their mixtures.

10. Nanoemulsion according to any one of the preceding claims, **characterized in that** the oily phase additionally comprises at least one oil having a molecular weight of less than 400.

11. Nanoemulsion according to any one of the preceding claims, **characterized in that** the oily phase comprises at least 40% by weight of oil(s) having a molecular weight of greater than 400 with respect to the total weight of the oily phase.

12. Nanoemulsion according to any one of the preceding claims, **characterized in that** the amount of oily phase ranges from 2 to 40% by weight with respect to the total weight of the nanoemulsion

13. Nanoemulsion according to any one of the preceding claims, **characterized in that** it additionally comprises at least one ionic amphiphilic lipid chosen from the group formed by anionic amphiphilic lipids and alkylsulphonic derivatives.

14. Nanoemulsion according to Claim 13, **characterized in that** the ionic amphiphilic lipids are chosen from the group formed by:
- the alkaline salts of dicetyl and dimyristyl phosphate;
- the alkaline salts of cholesterol sulphate;
- the alkaline salts of cholesterol phosphate;
- the salts of lipoamino acids;
- the sodium salts of phosphatidic acid;
- phospholipids;
- the alkylsulphonic derivatives of formula (I); 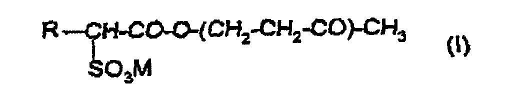 in which R represents C₁₆-C₂₂ alkyl radicals, taken as a mixture or separately, and M is an alkali metal; and their mixtures.

15. Nanoemulsion according to Claim 13 or 14, **characterized in that** the amount of ionic amphiphilic lipid(s) ranges from 0.01 to 5% by weight with respect to the total weight of the nanoemulsion.

16. Nanoemulsion according to any one of the preceding claims, **characterized in that** it comprises an additive which makes it possible to improve the transparency chosen from lower alcohols, glycols, sugars and their mixtures.

17. Nanoemulsion according to the preceding claim, **characterized in that** the additive is present in a concentration ranging from 5 to 20% by weight with respect to the total weight of the nanoemulsion.

18. Nanoemulsion according to any one of the preceding claims, **characterized in that** it comprises a cosmetic, dermatological or ophthaimological active principle.

19. Composition for topical use, **characterized in that** it comprises a nanoemulsion according to any one of Claims 1 to 18.

20. Ophthalmic support, **characterised in that** it comprises a nanoemulsion according to any one of Claims 1 to 18.

21. Pharmaceutical composition, **characterized in that** it comprises a nanemulsion according to any one of Claims 1 to 18.

22. Cosmetic use of the nanoemulsion according to any one of Claims 1 to 18 for caring for, treating and/or making up the skin, face and/or scalp.

23. Cosmetic use of the nanoemulsion according to any one of Claims 1 to 18 for caring for and/or treating the hair.

24. Cosmetic process for caring for and/or moisturizing the skin, mucous membranes and/or scalp, **characterized in that** a nanoemulsion according to any one of Claims 1 to 18 is applied to the skin, mucous membranes and/or scalp.

25. Use of the nanoemulsion according to any one of Claims 1 to 18 in the manufacture of a dermatological composition intended for the treatment of dry skin.

26. Use of the nanoemulsion according to any one of Claims 1 to 18 in the manufacture of an ophthalmological composition.

27. Process for the preparation of a nanoemulsion according to any one of Claims 1 to 18 which comprises the mixing of the aqueous phase and the oily phase with vigorous stirring at an ambient temperature ranging from 10 to 80°C and then a homogenization of the mixture at a pressure ranging from 6 × 10⁷ Pa to 18 × 10⁷ Pa.

28. Process according to the preceding claim, **characterized in that** the shearing ranges from 2 × 10⁶ s⁻¹ to 5 × 10⁸ s⁻¹.
